# EUROPEAN PATENT APPLICATION

(11) **EP 0 894 501 A1**
(43) Date of publication of application: **03.02.1999**
(21) Application number: 97917442.2
(22) Date of filing: 18.04.1997
(51) Int. Cl.: A61K 39/395, C07K 16/28, C12P 21/08

(54) **RHEUMATOID ARTHRITIS REMEDY CONTAINING ANTI-IL-8 ANTIBODY AS ACTIVE INGREDIENT**

(30) Priority: 19.04.1996 JP 98776/96
(71) Applicant: Chugai Seiyaku Kabushiki Kaisha, Tokyo 115 (JP)
(72) Inventor: AKAHOSHI, Tohru, Machida-shi, Tokyo 195 (JP); MATSUSHIMA, Kouji, Kanazawa-shi, Ishikawa 921 (JP)
(74) Representative: Vleck, Jan Montagu
(86) International application number: JP9701358
(87) International publication number: WO9739772

(57) **Abstract**

A rheumatoid arthritis remedy containing an anti-IL-8 antibody as the active ingredient.

## Description

### Technical Field

The present invention relates to a therapeutic agent for treating rheumatoid arthritis comprising anti-interleukin-8 (IL-8) antibody as an active ingredient.

### Background Art

IL-8 is a protein that belongs to the C-X-C chemokine subfamily and was formerly designated as the monocyte-derived neutrophil chemotactic factor, the neutrophil attractant/activation protein-1, the neutrophil activating factor and the like. IL-8 is a factor that activates neutrophils and provides them with migratory ability, and is produced by a variety of cells in the presence of inflammatory cytokines such as IL-1β, TNFα, etc. (Koch, A.E. et al., J. Investig. Med. (1995) 43, 28-38; Larsen, C.G. et al., Immunology (1989) 68, 31-36), mitogens such as PMA, LPS etc. (Yoshimura, T. et al, Proc. Natl. Acad. Sci. U.S.A. (1987) 84, 9233-9237), and heavy metals such as cadmium etc. (Horiguchi, H. et al., Lymphokine Cytokine Res. (1993) 12, 421-428).

It was reported that monocytes isolated from the synovial fluid of patients with rheumatoid arthritis had the elevated levels of expression of chemokines such as IL-8, GRO, MCAF, MIP-1α, MIP-1β and the like as compared to the monocytes isolated from the peripheral blood of healthy subjects or patients with rheumatoid arthritis (Hosaka, S. et al., Clin. Exp. Immunol. (1994) 97, 451-457). It is believed that overexpression of those various cytokines promotes migration of inflammatory cells to the joints, but it is not known which chemokine plays a central role in the pathogenesis.

An in vitro experiment suggests the possibility that IL-8 might be involved in the pathogenesis of rheumatoid arthritis because it activates neutrophils and induces cartilage destruction (Elford, P.R. and Cooper, P.H., Arthritis Rheum. (1991) 34, 325-332). Whereas another paper reported that there was no correlation between the levels of chemotactic activity and the concentration of IL-8 in the synovial fluids collected from patients with rheumatoid arthritis, and therefore the disease might be caused by other factors having the neutrophil chemotactic activity (Brennan, F.M. et al., Eur. J. Immunol. (1990) 20, 2141-2144).

It is known that an anti-IL-8 antibody is effective in vivo against experimental acute arthritis in an animal model. Furthermore, it was suggested that IL-8 antagonists IL-8 production inhibitors might be useful for the treatment for inflammatory diseases related to leukocyte infiltration including acute and chronic arthritis (Akahoshi, T. et al., Lymphokine Cytokine Res. (1994) 13, 113-116). However, it has not been known at all that anti-IL-8 antibody has a therapeutic effect against rheumatoid arthritis.

### Disclosure of the Invention

Anti-rheumatoid drugs, nonsteroidal anti-inflammatory drugs, and steroids have been used for the treatment for rheumatoid arthritis, so far, however, it is now known that the prolonged use of these drugs may induce undesirable side effects such as gastrointestinal disorders, skin eruption, renal disorders, osteoporosis and the like. Therefore, there has long been a need for the development of therapeutic agents for treating rheumatoid arthritis, which have small side effects. It is an object of the present invention to provide a therapeutic agent for treating rheumatoid arthritis, which has no disadvantages as mentioned above.

As a result of an intensive study to attain the above-mentioned objects, the applicants have found that anti-IL-8 antibody is useful as a therapeutic agent for rheumatoid arthritis and thereby have completed the present invention.

Accordingly, the present invention provides a therapeutic agent for rheumatoid arthritis which comprises anti-IL-8 antibody as an active ingredient.

### Embodiment for Carrying Out the Invention

### 1. Anti-IL-8 antibodies

Anti-IL-8 antibodies for use in the present invention may be of any origin, any kind (monoclonal or polyclonal), and any form, as long as they have a therapeutic effect against rheumatoid arthritis.

Anti-IL-8 antibodies for use in the present invention can be obtained as polyclonal or monoclonal antibodies using known methods. As the anti-IL-8 antibodies for use in the present invention, monoclonal antibodies of, in particular, mammalian origin, are preferred. Monoclonal antibodies of mammalian origin include those produced by hybridomas or hosts which have been transformed with expression vectors containing genetically engineered antibody genes. The antibodies bind to IL-8 to block the binding of IL-8 to IL-8 receptors expressed on neutrophils etc. and thereby inhibit the signal transduction of IL-8, and therefore the antibodies inhibit the biological activity of IL-8.

Examples of such antibodies include WS-4 antibody (Ko, Y. et al., J. Immunol. Methods (1992) 149, 227-235) and DM/C7 antibody (Mulligan, M.S. et al., J. Immunol. (1993) 159, 5585-5595), Pep-1 antibody and Pep-3 antibody (International Patent Application WO 92/04372), or 6G4.2.5 antibody and A5.12.14 antibody (International Patent Application WO 95/23865; Boylan, A.M. et al., J. Clin. Invest. (1992) 89, 1257-1267) etc. Among them, WS-4 antibody is especially preferred.

Incidentally, the hybridoma cell line which produces WS-4 antibody has been internationally deposited under the provisions of the Budapest Treaty as mouse hybridoma WS-4 on April 17, 1996 with the National Institute of Bioscience and Human Technology, Agency of Industrial Science and Technology, of 1-3, Higashi 1-chome, Tsukuba-shi, Ibaraki, Japan, as FERM BP-5507.

### 2. Antibody-producing hybridomas

Hybridomas producing monoclonal antibodies can be basically constructed using known procedures as described bellow. Thus, IL-8 is used as a sensitizing antigen. Hosts are immunized with IL-8 by a conventional method of immunization. The immune cells are collected to be fused with known parent cells by a conventional cell fusion method. And then hybridomas producing monoclonal antibodies are selected out of them by a conventional screening method to screen monoclonal antibody-producing cells.

More specifically, monoclonal antibodies may be obtained in the following manner. For example, IL-8 used as the sensitizing antigen for inducing antibodies can be obtained using the respective IL-8 gene/amino acid sequences as disclosed in Matsushima, K. et al., J. Exp. Med. (1988) 167, 1883-1893 for human IL-8, in Yoshimura, T. and Johnson, D.G., J. Immunol. (1993) 151, 6225-6236 for guinea pig IL-8, in Goodman, R.B. et al., Biochemistry (1992) 31, 10483-10490 for porcine IL-8, in Harada, A. et al., Int. Immunol. (1993) 5, 681-690 for rabbit IL-8, in Ishikawa, J. et al., Gene (1993) 131, 305-306 for canine IL-8, Seow, H.F. et al., Immunol. Cell Biol. (1994) 72, 398-405 for sheep IL-8, Villinger, F. et al, J. Immunol. (1995) 155, 3946-3954 for simian IL-8.

It is known that human IL-8 is produced in a variety of cells and undergoes various processing at the N-terminal end(Leonard, E.J. et al., Am. J. Respir. Cell. Mol. Biol. (1990) 2, 479-486). Though IL-8 that have 79, 77, 72, 71, 70 or 69 amino acid residues has been known so far, the number of amino acid residues is not limited in any way so long as the IL-8 can be used as the antigen for inducing anti-IL-8 antibodies to be used in the present invention. After placing one of these IL-8 genes into known expression vectors and transforming appropriate host cells with the vectors, IL-8 protein can be purified from the host cells or their culture supernatants using known procedures. The purified IL-8 may be used as an sensitizing antigen.

Preferably mammals to be immunized with the sensitizing antigen are selected in consideration of their compatibility with the parent cells for use in cell fusion generally and they generally include, but not limited to, rodents such as a mouse, rat, hamster, and the like.

Immunization of animals with a sensitizing antigen is carried out using known methods. General methods, for example, includes intraperitoneal or subcutaneous administration of the sensitization antigen to one or more mammals. Specifically, the sensitization antigen, which is diluted and suspended in an appropriate amount of phosphate buffered saline (PBS) or physiological saline etc. is mixed with an appropriate amount of adjuvant, such as Freund's complete adjuvant, at the request. After being emulsified, it is preferably administered to one or more mammals for several times every 4 to 21 days. Additionally suitable carriers may be used at the time of immunization with the sensitizing antigen.

After the immunization and confirmation of the increase in the desired antibody levels in the serum by a conventional method, the immune cells are taken out from the mammal and are subjected to cell fusion, in which preferred immune cells include in particular the spleen cells.

The mammalian myeloma cells as the parent cells which are subjected to cell fusion with the above-mentioned immune cells, preferably include various known cell lines such as P3 (P3x63Ag8.653) (Kearney, J.F. et al., J. Immunol. (1979) 123, 1548-1550), P3x63Ag8U1 (Yelton, D.E. et al., Current Topics in Microbiology and Immunology (1978) 81, 1-7), NS-1 (Kohler, G. and Milstein, C., Eur. J. Immunol. (1976) 6, 511-519), MPC-11 (Margulies, D.H. et al., Cell (1976) 8, 405-415), SP2/0 (Shulman, M. et al., Nature (1978) 276, 269-270), F0 (de St. Groth, S.F. and Scheidegger, D., J. Immunol. Methods (1980) 35, 1-21), S194 (Trowbridge, I.S., J. Exp. Med. (1978) 148, 313-323), R210 (Galfre, G. et al., Nature (1979) 277, 131-133) and the like.

Cell fusion between the above immune cells and the myeloma cells may be essentially performed by known methods such as is described in Milstein et al. (Galfre, G. and Milsterin, C., Methods Enzymol. (1981) 73, 3-46) and the like.

More specifically, the above cell fusion is carried out in a conventional nutrient medium in the presence of, for example, a cell fusion accelerator. As the cell fusion accelerator, for example, polyethylene glycol (PEG), Sendai virus (HVJ) and the like may be used, and an assistant agent such as dimethyl sulfoxide etc. may be added as desired to enhance efficiency of the fusion.

The preferred ratio of the immune cells and the myeloma cells to be used is, for example, 1 to 10 times more immune cells than the myeloma cells. Examples of culture media to be used for the above cell fusion include RPMI 1640 medium and MEM culture medium suitable for the growth of the above myeloma cell lines, and the conventional culture medium used for this type of cell culture. Besides, serum supplements such as fetal calf serum (FCS) may be added to them.

In cell fusion, predetermined amounts of the above immune cells and the myeloma cells are mixed well in the above culture medium, to which a PEG solution, for example the PEG solution with a mean molecular weight of 1000 to 6000, previously heated to about 37 °C is added at a concentration of 30 to 60% (w/v) and mixed to obtain the desired fusion cells (hybridomas). Then by repeating the sequential addition of a suitable culture liquid and centrifugation to remove the supernatant, cell fusion agents etc. which are undesirable for the growth of the hybridoma can be removed.

The hybridoma is selected by culturing in a conventional selection medium, for example, HAT culture medium (a culture liquid containing hypoxanthine, aminopterin, and thymidine). Culturing in the HAT culture medium is continued generally for the period of time sufficient to annihilate the cells other than the desired hybridomas (non-fusion cells), generally several days to several weeks. A conventional limiting dilution method is performed in which the hybridomas producing the desired antibody are screened and monoclonally cloned.

In addition to obtaining the above hybridoma by immunizing non-human animals with the antigen, it is also possible to immunize human lymphocytes in vitro with IL-8, and the resulting sensitized lymphocytes are fused with myeloma cells, for example U266, having the ability of dividing permanently to obtain the desired human antibodies having the activity of binding to IL-8 (Japanese Post-examined Patent Publication (Kokoku) 1-59878). Furthermore, it is possible that a transgenic animals having a repertoire of human antibody genes are immunized with the antigen IL-8 to obtain anti-IL-8 antibody-producing cells. Then the cells are immortalized and used to obtain human antibodies to IL-8 (International Patent Application WO 92/03918, WO 93/12227, WO 94/02602, WO 94/25585, WO 96/33735 and WO 96/34096).

The monoclonal antibody-producing hybridomas thus constructed can be subcultured in the conventional culture medium, or can be stored for a prolonged period of time in liquid nitrogen.

In order to obtain monoclonal antibodies from the hybridomas, there can be mentioned a method in which the hybridomas are cultured by conventional methods and the antibodies are obtained as the supernatant, or a method in which the hybridoma is transplanted to and grown in a mammals compatible with them and the antibodies are obtained in the ascites. The former method is suitable for obtaining high-purity antibodies, whereas the latter is suitable for large scale production of antibodies.

### 3. Recombinant antibodies

Recombinant antibodies, which are produced by the recombinant gene technology in which antibody genes are cloned from the hybridomas and placed into suitable vectors to be then transfected into host cells, can be used in the present invention as monoclonal antibodies (for example, Borrebaeck, C.A.K. and Larrick, J.W., THERAPEUTIC MONOCLONAL ANTIBODIES, published in the United Kingdom by MACMILLAN PUBLISHERS LTD. 1990).

Specifically, mRNA encoding the variable region (V region) of anti-IL-8 antibody is isolated from the hybridoma producing anti-IL-8 antibody. The isolation of mRNA is conducted by preparing total RNA using, for example, a known method such as the guanidine ultracentrifugation method (Chirgwin, J.M. et al., Biochemistry (1979) 18, 5294-5299), the AGPC method (Chomczynski, P. and Sacchi, N., Anal. Biochem. (1987) 162, 156-159) and the like, and then mRNA is purified from the total RNA using an mRNA Purification kit (Pharmacia) and the like. Alternatively, mRNA can be directly prepared using a Quick Prep mRNA Purification Kit (Pharmacia).

cDNA of the V region of antibody may be synthesized from the mRNA thus obtained using a reverse transcriptase. cDNA may be synthesized using an AMV Reverse Transcriptase First-strand cDNA Synthesis Kit (Seikagaku Kogyo), and the like. Alternatively, for the synthesis and amplification of cDNA, a 5'-RACE method (Frohman, M.A. et al., Proc. Natl. Acad. Sci. U.S.A. (1988) 85, 8998-9002; Belyavsky, A. et al., Nucleic Acids Res. (1989) 17, 2919-2932) using the 5'-Ampli FINDER RACE Kit (Clontech) and the polymerase chain reaction (PCR) may be used.

The desired DNA fragments are purified from the obtained PCR products and are ligated to respective vector DNAs. Sequentially, recombinant vectors can be constructed therefrom and then be transfected into E. coli etc. The transfectant colonies can be selected to prepare the desired recombinant vector. The nucleotide sequences of the desired recombinant DNA may be confirmed by known methods such as the dideoxy nucleotide chain termination method.

Once the DNA encoding the V regions of the desired anti-IL-8 antibody have been obtained, they may be ligated to DNA encoding the constant regions (C regions) of the other antibodies, and be then placed into respective expression vectors. Alternatively, the DNA encoding the V regions of the antibody may be placed into respective expression vectors which already contain DNA encoding the C regions of the antibodies.

In order to produce anti-IL-8 antibodies for use in the present invention, the antibody genes are placed into expression vectors so as to be expressed under the control of the expression regulatory regions, for example an enhancer and/or a promoter. Subsequently, with the expression vectors, host cells are transformed and the desired antibodies are then expressed therein.

Expression of antibody genes may be attained by transforming host cells simultaneously with two sets of expression vectors into which DNA encoding the heavy chain (H chain) and the light chain (L chain) of the antibody are respectively placed. Alternatively, it may be attained by transforming host cells with single expression vectors into which DNA encoding both the H chain and the L chain are placed (International Patent Application WO 94/11523).

### 4. Altered antibodies

In the present invention, artificially altered recombinant antibodies such as chimeric antibodies and humanized antibodies can be used for the purpose of lowering heterologous antigenicity against human. These altered antibodies can be produced using known methods.

Chimeric antibodies can be obtained as follows. The obtained DNA encoding the V regions of non-human antibodies are ligated to DNA encoding the C regions of human antibodies. The resulting DNA fragments are placed into expression vectors. The vectors are transfected into hosts, and chimeric antibodies are produced therein (European Patent Application EP 125023, and International Patent Application WO 96/02576). Using such known methods, chimeric antibodies useful for the present invention can be obtained.

E. coli having the plasmid coding for L chain or H chain of chimeric WS-4 antibody has been internationally deposited under the provisions of the Budapest Treaty as Escherichia coli DH5α (HEF-chWS4L-gκ) and Escherichia coli

JM109 (HEF-chWS4H-gγ1) on July 12, 1994 with the National Institute of Bioscience and Human Technology Agency of Industrial Science and Technology, of 1-3, Higashi 1-chome, Tsukuba-shi, Ibaraki pref., Japan, as FERM BP-4739 and FERM BP-4740, respectively. Humanized antibody which is also called reshaped human antibody has been produced by transplanting complementarity determining regions (CDRs) of antibody of a mammal other than the human, for example mouse antibody, into the CDRs of human antibody. The general recombinant DNA technology for preparation of such antibodies is also known (see European Patent Application EP 125023 and International Patent Application WO 96/02576).

Specifically, DNA sequences which are designed to ligate the CDRs of a mouse antibody to framework regions (FRs) of human one or more antibodies are synthesized. Briefly, several divided oligonucleotides having sections overlapping with one another at the ends thereof are synthesized, and then, single DNA fragments are synthesized using PCR methods. The DNA fragments thus obtained are ligated to DNA encoding the C regions of human antibody and then are placed into expression vectors, which are transfected into hosts for antibody production (European Patent Application EP 239400 and International Patent Application WO 96/02576).

The FRs of one or more human antibodies being ligated to CDRs are selected to form favorable antigen-binding sites. When desired, amino acids in the FRs of antibody V region may be substituted so that the CDRs of humanized antibody may form an appropriate antigen biding site (Sato, K. et al., Cancer Res. (1993) 53, 851-856).

For chimeric antibodies and humanized antibodies, the C regions of human antibody may be used depending on the purpose. For example, Cγ1, Cγ2, Cγ3, and Cγ4 can be used. The C regions of human antibody may also be modified in order to improve the stability of the antibodies and of the production thereof. For example, when the subclass IgG4 of antibody is chosen, the amino acid sequence CPSCP of part of the hinge region of IgG4 can be converted to the amino acid sequence CPPCP of the hinge region of IgG1 to resolve the structural instability of IgG4 (Angal, S. et al., Mol. Immunol. (1993) 30, 105-108).

Chimeric antibodies comprise the V regions of non-human antibody and the C regions of human antibody, whereas humanized antibodies comprise the CDRs of non-human mammal antibody and the FRs and the C region of one or more human antibodies. Accordingly, since the amino acid residues derived from non-human mammals other than the human are reduced to a minimum in the above antibodies. That is, the antigenicity thereof in the human body is reduced so that they are useful as an active ingredient in the therapeutic agents for use in the present invention.

A preferred embodiment of humanized antibody for use in the present invention includes humanized WS-4 antibody (International Patent Application WO 96/02576). In the humanized WS-4 antibody, CDRs of the WS-4 antibody derived from a mouse have been ligated to the FRs of the human antibody REI for the L chain, and the FR1-3 of the human antibody VDH26 and the FR4 of the human antibody 4B4 for the H chain, and part of the amino acid residues of the FR has been substituted to obtain antigen-binding activity.

E. coli having the plasmid coding for the L chain or the H chain of humanized WS-4 antibody has been deposited under the provisions of the Budapest Treaty as Escherichia coli DH5α (HEF-RVLa-gκ) and Escherichia coli JM109 (HEF-RVHg-gγ1) on July 12, 1994 with the National Institute of Bioscience and Human Technology, Agency of Industrial Science and Technology, of 1-3, Higashi 1-chome, Tsukuba-shi, Ibaraki, Japan, as FERM BP-4738 and FERM BP-4741, respectively.

### 5. Modified antibodies

Antibodies for use in the present invention may include fragments of antibody and modified versions thereof as long as they bind to IL-8 and thereby inhibit the activity of IL-8. For example, as fragments of antibody, there may be mentioned Fab, F(ab')2, Fv or single-chain Fv (scFv) in which Fv's of an H chain and an L chain of Fv have been ligated via a suitable linker. Specifically antibodies are treated with an enzyme, for example, papain or pepsin, to produce antibody fragments, or genes encoding these antibody fragments are constructed, and then placed into expression vectors, to be expressed in suitable host cells (see, for example, Co, M.S. et al., J. Immunol. (1994) 152, 2968-2976; Better, M. and Horwitz, A.H., Methods Enzymol. (1989) 178, 476-496; Pluckthun, A. and Skerra, A., Methods Enzymol. (1989) 178, 497-515; Lamoyi, E., Methods Enzymol. (1986) 121, 652-663; Rousseaux, J. et al., Methods Enzymol. (1986) 121, 663-669; Bird, R.E. and Walker, B. W., Trends Biotechnol. (1991) 9, 132-137).

scFv can be obtained by ligating a V region of H chain and that of L chain of antibody. In the scFv, the V region of H chain and that of L chain are ligated via a linker, preferably a peptide linker (Huston, J.S. et al., Proc. Natl. Acad. Sci. U.S.A. (1988) 85, 5879-5883). The V region of an H chain and that of L chain in scFv may be derived from any of the above-mentioned antibodies. As peptide linkers for linking the V regions, any single-chain peptide, for example, that comprising 12 - 19 amino acid residues may be used.

DNA encoding scFv can be obtained using DNA encoding an H chain or an H chain V region of the above antibody and DNA encoding an L chain or an L chain V region of the above antibody as templates for amplifying a portion of these template DNA, which encode the required amino acid sequences by the PCR technique with the primer pair specifying the both ends thereof. Using combined amplifying methods with these DNA portions, DNA encoding a peptide linker portion and the primer pair defining its both ends to be ligated to the H chain and L chain, respectively, the desired DNA encoding scFv can be obtained.

Once DNA encoding scFv is constructed, an expression vector containing it and a host transformed with said expression vector can be obtained by the conventional methods, and scFv can be obtained using the resultant host by the conventional methods.

These antibody fragments can be produced by obtaining the gene thereof in a similar manner to that mentioned above and by allowing it to be expressed in a host. "Antibody" as used in the claim of the present application encompasses these antibody fragments.

As modified antibodies, anti-IL-8 antibody conjugated to various molecules such as polyethylene glycol (PEG) can be used. "Antibody" as used in the claim of the present application encompasses these modified antibodies. These modified antibodies can be obtained by chemically modifying the antibodies thus obtained. These methods have already been established in the art.

### 6. Expression and production of recombinant antibodies and altered antibodies

Antibody genes constructed as mentioned above may be expressed and obtained by known methods. In the case of mammalian cells, the expression may be accomplished using expression vectors containing operably connected DNA which comprises conventional promoters, antibody genes to be expressed, and the poly A signal linked at 3' downstream thereof. Examples of the promoter/enhancer include human cytomegalovirus immediate early promoter/enhancer.

Additionally, as the promoter/enhancer which can be used for expression of antibodies for use in the present invention, there can be used viral promoters/enhancers such as retrovirus, polyoma virus, adenovirus, and simian virus 40 (SV40), and those derived from mammalian cells such as human elongation factor 1α (HEF1α). For example, expression may be readily accomplished by the method of Mulligan, R.C. et al. (Nature (1979) 277, 108-114) when SV40 promoter/enhancer is used, or by the method of Mizushima, S. et al. (Nucleic Acids Res. (1990) 18, 5322) when HEF1α promoter/enhancer is used.

In the case of E. coli, the expression may be accomplished by operably linking one or more conventional promoters, one or more signal sequences for antibody secretion, and one or more antibody genes to be expressed. As the promoters, for example, there can be mentioned lacz promoter and araB promoter. The method of Ward, E.S. et al. (Nature (1989) 341, 544-546; FASEB J. (1992) 6, 2422-2427) may be used when lacz promoter is used, and the method of Better, M. et al. (Science (1988) 240, 1041-1043) may be used when araB promoter is used.

As signal sequences for antibody secretion, when produced in the periplasm of E. coli, the pelB signal sequence (Lei, S.P. et al., J. Bacteriol. (1987) 169, 4379-4383) can be used. After isolating the antibody produced in the periplasm, the structure of the antibody is appropriately refolded before use (for example, International Patent Application WO 96/30394).

As the origin of replication, there can be used the ones derived from SV40, polyoma virus, adenovirus, bovine papilloma virus (BPV), and the like. Furthermore, for amplification of the gene copy number in the host cell system, expression vectors can contain the aminoglycoside transferase (APH) gene, the thymidine kinase (TK) gene, E. coli xanthine guaninephosphoribosyl transferase (Ecogpt) gene, the dihydrofolate reductase (dhfr) gene, or the like as selectable markers.

For the production of antibody for use in the present invention, any production system can be used, and the production systems of antibody preparation comprise in vitro and in vivo production system.

As the in vitro production systems, there can be mentioned production systems which employ eukaryotic cells and those which employ prokaryotic cells.

When the eukaryotic cells are used, there are production systems which employ animal cells, plant cells, or fungal cells. Known animal cells include (1) mammalian cells such as CHO cells, COS cells, myeloma cells, baby hamster kidney (BHK) cells, HeLa cells, and Vero cells, (2) amphibian cells such as Xenopus oocytes, and (3) insect cells such as Sf9, Sf21, and Tn5. Known plant cells include, for example, those derived from the Nicotiana family, more specifically, cells derived from Nicotiana tabacum which are subjected to callus culture. Known fungal cells include (1) yeasts such as the Saccharomyces family, more specifically, Saccharomyces cereviceae, and (2) filamentous fungi such as the Aspergillus family, more specifically, Aspergillus niger.

When the prokaryotic cells are used, there are the production systems which employ bacterial cells. Known bacterial cells include Escherichia coli, and Bacillus subtilis.

By transfecting desired antibody genes into these cells via transformation and culturing the transformed cells in vitro, the antibodies can be obtained. Culturing is performed by known methods. For example, as culture media for mammalian cells, DMEM, MEM, RPMI1640, IMDM and the like can be used, and serum supplements such as fetal calf serum (FCS) may be used in combination. In addition, antibodies may be produced in vivo by implanting cells into which the antibody genes are placed into the abdominal cavity of animals, and the like.

As other in vivo production systems, there can be mentioned those which employ animals and those which employ plants. When animals are used, there are production systems which employ mammals and insects.

As mammals, goats, pigs, sheep, mice, or cattle can be used (Glazer, V., SPECTRUM Biotechnology Applications, 1993). Also as insects, silkworms can be used.

When plants are used, tobacco, for example, can be used.

Antibody genes are placed into these animals or plants, and antibodies are produced in such animals or plants, and collected. For example, antibody genes are placed into the middle of the genes encoding proteins such as goat casein which are intrinsically produced in the milk to prepare fusion genes. DNA fragments containing the fusion genes into which the antibody genes are placed, are injected to goat embryos, and the embryos are implanted into female goats. The desired antibody is obtained from the milk produced by the transgenic goat borne to the goat who received the embryo or offsprings thereof. In order to increase the amount of milk containing the desired antibody produced by the transgenic goat, hormones may be given to the transgenic goat as appropriate. (Ebert, K.M. et al., Bio/Technology (1994) 12, 699-702).

When silkworms are used, they are infected with baculovirus into which desired antibody genes are placed, and the desired antibody can be obtained from their body fluid (Maeda, S. et al., Nature (1985) 315, 592-594). Moreover, when tobaccos are used, desired antibody genes are placed into expression vectors for plants, for example pMON 530, and then the vectors are transfected into bacteria such as Agrobacterium tumefaciens. With the bacteria tobaccos such as Nicotiana tabacum are then infected to obtain the desired antibodies from their leaves (Ma, J.K. et al., Eur. J. Immunol. (1994) 24, 131-138).

When antibodies are produced in in vitro or in vivo production systems as mentioned above, DNA encoding an H chain and that encoding L chain of antibody can be separately transfected into espective expression vectors and the hosts can be transformed by them simultaneously. Alternatively DNA encoding both H chain and L chain of antibody can be placed into one sort of expression vectors and hosts can be transformed thereby (International Patent Application WO 94/11523).

### 7. Isolation and purification of antibodies

Antibodies expressed and produced as described above can be isolated from inside or outside of the cells or from the hosts and then may be purified to homogeneity. Isolation and purification of antibodies for use in the present invention may be performed by affinity chromatography. As the columns used for such affinity chromatography, there can be mentioned Protein A columns and Protein G columns. Examples of the columns employing Protein A are Hyper D, POROS, Sepharose F.F. (Pharmacia) and the like. Alternatively, methods for isolation and purification conventionally used for proteins, can be used without any limitation. For example, isolation and purification of antibodies may be accomplished by combining, as appropriate, chromatography columns other than the above-mentioned affinity chromatography, filters, ultracentrifugation, salting-out, dialysis and the like (Antibodies: A Laboratory Manual, Ed Harlow and David Lane, Cold Spring Harbor Laboratory, 1988). Chromatography other than affinity chromatography includes, for example, ion exchange chromatography, hydrophobic chromatography, gel-filtration and the like (Strategies for Protein Purification and Characterization: A Laboratory Course Manual, Ed Daniel R. Marshak et al., Cold Spring Harbor Laboratory Press, 1996).

### 8. Measurement of antibody concentration

The concentration of antibodies obtained in the above 7 can be determined by measurement of absorbance or by the enzyme-linked immunosorbent assay (ELISA) and the like. Briefly, when absorbance measurement is employed, the antibodies obtained are appropriately diluted with PBS and then the absorbance is measured at 280 nm, followed by calculation using the absorption coefficient which is, 1.4 OD at 1 mg/ml in the case of human antibody and different among different species and subclasses. When the ELISA method is used, measurement is performed as follows. Thus, 100 µl of goat anti-human IgG antibody diluted to 1 µg/ml in 0.1 M bicarbonate buffer, pH 9.6, is added to 96-well plates (Nunc), followed by incubating overnight at 4 °C to immobilize the antibody. After blocking, 100 µl each of appropriately diluted antibody in the present invention or samples containing the antibodies, and 100 µl of human IgG of known concentrations as the concentration standard, are added, followed by incubating at room temperature for 1 hour. After washing, 100 µl of 5000-fold diluted alkaline phosphatase-labeled anti-human IgG antibody is added, followed by incubating at room temperature for 1 hour. After washing, the substrate solution is added, followed by incubation and measurement of absorbance at 405 nm using the MICROPLATE READER Model 3550 (Bio-Rad) to calculate the concentration of the desired antibody based on the absorbance of the concentration standard IgG.

### 9. Confirmation of the activity of antibodies

Known methods can be used for the measurement of the antigen-binding activity (Antibodies: A Laboratory Manual. Ed Harlow and David Lane, Cold Spring Harbor Laboratory, 1988) and the ligand/receptor binding inhibition activity (Harada, A. et al., Int. Immunol. (1995) 5, 681-690) of the antibodies used in the present invention.

As methods for determining the antigen-binding activity of anti-IL-8 antibodies for use in the present invention, there can be used ELISA, EIA (enzymeimmunoassay), RIA (radioimmunoassay), or the fluorescent antibody method. When ELISA is employed, for example, IL-8 is added to 96-well plates onto which polyclonal antibody against IL-8 has been immobilized, and then samples containing the desired anti-IL-8 antibody, for example culture supernatants of anti-IL-8 antibody-producing cells or purified antibody, are added thereto. Secondary antibody labeled with an enzyme such as alkaline phosphatase, which recognizes the desired anti-IL-8 antibody, is added, and the plates are incubated, washed, and then the enzyme substrate is added, and the absorbance is measured to evaluate the antigen-binding activity.

As methods for measuring the inhibition activity of the anti-IL-8 antibodies against ligand/receptor binding for use in the present invention, the conventional Cell ELISA or the ligand receptor binding assay can be used.

In the case of Cell ELISA, for example, blood cells or cancer cells expressing IL-8 receptors such as neutrophils are cultured in 96-well plates to allow the cells to adhere thereonto, which are then immobilized with paraformaldehyde etc. Alternatively, the membrane fraction of cells expressing IL-8 receptors is prepared and 96-well plates on which the fraction has been immobilized are prepared.

Onto these plates, samples containing the desired anti-IL-8 antibody, for example culture supernatants of anti-IL-8 antibody-producing cells and purified antibody, and a radioisotope such as ¹²⁵I-labeled IL-8, are added. After incubating and washing the plates, radioactivity is measured to determine the amount of IL-8 bound to IL-8 receptors and thereby to evaluate the inhibiting activity of anti-IL-8 antibodies against ligand/receptor binding.

The inhibition assays of IL-8 binding to IL-8 receptors on the cells are performed as follows. Blood cells or cancer cells expressing IL-8 receptors such as neutrophils are isolated by means of centrifugation etc. to prepare cell suspension. A solution of IL-8 labeled with a radioisotope such as ¹²⁵I, or a mixture of unlabeled IL-8 and labeled IL-8, are added to the cell suspension together with a solution comprising anti-IL-8 antibody whose concentration has been adjusted. After incubating for a certain period of time, the cells are isolated, and the radioactivity of the labeled IL-8 bound onto the cell is measured.

As methods for measuring the neutrophil chemotaxis inhibiting ability of anti-IL-8 antibodies for use in the present invention, known methods such as the one described by Grob, P.M. et al. (J. Biol. Chem. (1990) 265, 8311-8316) can be used.

Specifically, they can be carried out using commercial chemotaxis chamber systems. After diluting anti-IL-8 antibody with culture medium such as RPMI 1640, DMEM, MEM, or IMDM, IL-8 is added thereto. The resulting mixture is dispensed into the lower chamber partitioned by filters. Subsequently, prepared cell suspension, for example neutrophil suspension, is added to the upper chamber and then allowed to stand for a certain period of time. Migrating cells will adhere to the lower-side surface of the filter attached to the chamber, and therefore the number of cells adhered thereto can be measured by methods using stain or fluorescent antibody etc. Alternatively, visual examination under the microscope or automatic measurement using counting devices can be employed.

### 10. Methods of administration and pharmaceutical preparation

Diagnostic signs of rheumatoid arthritis include morning stiffness, swelling of joints in the hand, symmetric joint swelling, multiple joint swelling, and the like (Mimori, T. et al., Shindan to Chiryo (Diagnosis and Therapy) Vol. 83, No. 7, 1995 (65) 1187-1190, Arnett, F.C. et al., Arthritis Rheum. (1988) 31, 315-324). Accordingly, animals which have artificially-produced swollen joints are useful as the experimental animal system for use in the experiments on therapeutic effects on rheumatoid arthritis.

Therapeutic agents that contain anti-IL-8 antibody as an active ingredient in the present invention may be administered, either systemically or locally, by parenteral routes, for example intravenous injection such as drip infusion, intramuscular injection, intraperitoneal injection, subcutaneous injection, injection into articular cavities, and the like. The methods of administration may be chosen, as appropriate, depending on the age and the conditions of the patients.

Therapeutic agents that contain anti-IL-8 antibody as an active ingredient in the present invention may be administered to patients suffering from a disease in an amount sufficient to treat the disease and the symptoms of complications thereof or to prevent them at least partially. For example, the effective dosage is chosen from the range of 0.01 mg to 1000 mg per kg of body weight per administration. Alternatively, the dosage in the range of 5 to 2000 mg/body per patient may be chosen. However, the dosage of therapeutic agents containing anti-IL-8 antibody in the present invention is not limited to these dosages.

The timing of administration may be after the establishment of diagnosis of rheumatoid arthritis or at the time when the disease is suspected from the conditions.

Therapeutic agents that contain anti-IL-8 antibody as the active ingredient in the present invention may be formulated into a pharmaceutical preparation (Remington's Pharmaceutical Science, latest edition, Mark Publishing Company, Easton, U.S.A.), and may further contain pharmaceutically acceptable carriers or additives.

Examples of such carriers or pharmaceutical additives include water, pharmaceutical acceptable organic solvent, collagen, polyvinyl alcohol, polyvinylpyrrolidone, carboxyvinyl polymers, sodium carboxymethylcellulose, sodium polyacrylic acid, sodium alginate, water-soluble dextran, sodium carboxymethyl starch, pectin, methyl cellulose, ethyl cellulose, xanthan gum, gum arabic, casein, agar, polyethylene glycol, diglycerin, glycerin, propylene glycol, vaseline, paraffin, stearyl alcohol, searic acid, human serum albumin (HSA), mannitol, sorbitol, lactose, pharmaceutically acceptable surfactants and the like.

Actual additives are chosen from, but not limited to, the above or combinations thereof depending on the dosage form.

When used as parenteral injections, purified anti-IL-8 antibodies may be dissolved in solvent, for example, physiological saline, buffers, glucose solution, etc., to which one or more anti-adsorption agents such as Tween 80, Tween 20, gelatin, human serum albumin etc. are added. Alternatively, lyophilized agents which are to be reconstituted prior to use, may be used. As an excipients for lyophilization, sugars such as mannitol, glucose etc. can be used.

### Examples

The present invention will now be explained hereinbelow in more detail with reference to the following reference examples and working examples. It is to be noted that the present invention is not limited to these examples in any way.

### Reference example 1. Construction of a hybridoma that produces monoclonal antibody against human IL-8

Human IL-8 is given to Balb/c mice according to the conventional method, and splenocytes were collected from the mice in which immunization was established. According to the conventional method which utilizes polyethylene glycol, the splenocytes were fused with the mouse myeloma P3x63Ag8.653 according to the conventional method to construct a hybridoma that produces monoclonal antibody against human IL-8. After screening based on the binding activity of the antibody to human IL-8 as an index, the hybridoma cell line WS-4 was obtained. The antibody produced by the hybridoma WS-4 had the activity of inhibiting the binding of IL-8 to neutrophils, i.e. neutralizing activity (Ko, Y. et al., J. Immunol. Methods (1992) 149, 227-235).

The isotypes of the H chain and the L chain of the antibody produced by the hybridoma WS-4 were examined using the mouse monoclonal antibody isotyping kit. The result revealed that the antibody produced by the hybridoma WS-4 has mouse κ type L chain and mouse γ1 type H chain.

The hybridoma cell line WS-4 was internationally deposited under the provisions of the Budapest Treaty on April 17, 1996 with the National Institute of Bioscience and Human Technology Agency of Industrial Science and Technology, of 1-3, Higashi 1-chome, Tsukuba-shi, Ibaraki., Japan, as FERM BP-5507.

### Reference example 2. Construction of humanized antibodies against human IL-8

The humanized WS-4 antibody was constructed as described in International Patent Application WO 96/02576.

From the hybridoma WS-4 prepared in Reference example 1, total RNA was prepared by the conventional method, and single-stranded cDNA was synthesized therefrom. By the PCR method, DNA encoding the V regions of H chain and L chain of the mouse WS-4 antibody were amplified. The primers used in the PCR method are those described in Jones, S.T. and Bendig, M.M., Bio/Technology (1991) 9, 88-99. The PCR-amplified DNA fragments were purified, and the DNA fragment containing the gene encoding the L chain V region of the mouse WS-4 antibody and the DNA fragment containing the gene encoding the H chain V region of the mouse WS-4 antibody were isolated. These DNA fragments were placed into respective pUC cloning vectors, which were then transfected into competent E. coli cells to obtain E. coli transformants.

The transformants were cultured by a conventional method, and plasmids containing the above DNA fragments were purified from the cell mass thus obtained. The base sequence of DNA encoding the V regions in the plasmid were determined by the conventional method, and the CDRs of each V region were identified from the amino acid sequences.

In order to construct vectors that express chimera WS-4 antibody, cDNA encoding the V region of L chain and H chain of mouse WS-4 antibody were separately placed into respective HEF vectors that were previously ligated to DNA encoding human C region.

In order to construct humanized WS-4 antibodies, genetic engineering technique based on the CDR grafting method was used to implant the CDRs of V regions of mouse WS-4 antibody to human antibody. In order to form appropriate antigen-binding sites, substitution of DNA sequences for partial substitution of amino acids in the FR of V regions of CDR-implanted antibody was conducted.

In order to express the V regions of L chain and H chain of humanized WS-4 antibody thus constructed as antibody in mammalian cells, DNA encoding each was separately placed into respective HEF vectors, and a vector expressing the L chain or the H chain of humanized WS-4 antibody was constructed.

By co-transfecting these two expression vectors into COS cells, cell lines that produce humanized WS-4 antibodies were established. The ability to bind to IL-8 and neutralize IL-8 of the humanized WS-4 antibody obtained by culturing the thus obtained cell line was evaluated by ELISA and the inhibition test of IL-8/neutrophil binding, respectively. The results revealed that the humanized WS-4 antibodies inhibit the binding of IL-8 to neutrophil by binding to human IL-8 in an extent similar to that of mouse WS-4 antibody.

E. coli having the plasmid containing the L chain or the H chain of humanized WS-4 antibody was internationally deposited under the provisions of the Budapest Treaty as Escherichia coli DH5α (HEF-RVLa-gκ) and Escherichia coli JM109 (HEF-RVHg-gγ1) on July 12, 1994 with the National Institute of Bioscience and Human Technology, Agency of Industrial Science and Technology, of 1-3, Higashi 1-chome, Tsukuba-shi, Ibaraki, Japan, as FERM BP-4738 and FERM BP-4741, respectively.

### Working example 1.

A solution of bovine serum albumin (BSA: Nacalai Tesque) dissolved in phosphate buffered saline (PBS: Nissui Pharmaceutical) at 10 mg/ml was mixed with an equal amount of Freund's complete adjuvant (Sigma) and was emulsified. The emulsion was injected to immunize Japanese white rabbits (n=6 per group, male, three-month old, Gokita Laboratory) at an amount of 2 ml per rabbit (0.4 ml each subcutaneously at two sites of the dorsal side, 0.5 ml each into the muscle of both femurs, and 0.1 ml each at the plantar of both hind feet). On day 21 after immunization, BSA solution at a concentration of 10 mg/ml (solvent is PBS) and Freund's incomplete adjuvant (Sigma) were mixed in equal amounts and emulsified, 0.4 ml of which was then injected subcutaneously at the dorsal side of the BSA-immunized rabbits.

Furthermore, 7 days later (28 days after immunization) a skin test was conducted to confirm the establishment of immunization. Thus, 10 µg of BSA was dissolved in 0.1 ml of PBS and injected subcutaneously into the inside of the right ear. When a red spot equal to or greater than 10 mm was formed three days later, it was considered positive. After confirming the establishment of immunization against BSA (31 after immunization), the sensitized rabbits were anesthetized with Nembutal (Dainippon Pharmaceutical, dosage 0.4 mg/kg body weight), and then 10 mg of mouse WS-4 antibody against human IL-8 or 10 mg of the control antibody, mouse P3.6.2.8.1 antibody, was diluted in physiological saline (Ohtsuka Pharmaceutical), three ml of which was injected to the animals through the ear vein.

Immediately after antibody administration, an antigen solution prepared by diluting 0.5 ml of the BSA solution (solvent is PBS) at 10 mg/ml with 1.5 ml of physiological saline was injected into the cavity of the left knee joint using a 23G needle. Subsequently, 24 hours later the animals were sacrificed by excessively anesthetizing (dosage 2.0 mg/kg body weight) with Nembutal, and then the diameters of both knee joints were measured at bilateral knee joint fissure sites with a caliper square. From the measurement, the difference in the diameter of knee joints between the left knee joint in which joint swelling was induced and the normal right knee joint was calculated to determine joint swelling. The results are shown in Table 1.

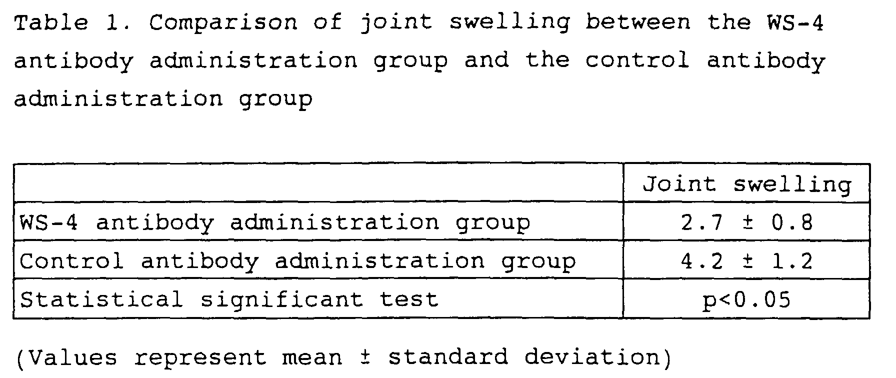

The mean difference in diameter between the right and the left knee joints was 4.2 +/- 1.2 mm in the control antibody administration group, whereas that in the WS-4 antibody administration group was 2.7 +/- 0.8 mm. Statistical analysis between the two groups with respect to the difference in the diameter between the right and the left knee joints revealed a significant difference. Thus, in the present example it was shown that WS-4 antibody significantly suppresses joint swelling. There has been no such reports so far that anti-IL-8 antibodies suppress joint swelling which is an index of the diagnostic criteria and therapeutic effect of rheumatoid arthritis, and this is demonstrated for the first time by the present invention.

## Claims

1. A therapeutic agent for treating rheumatoid arthritis comprising anti-IL-8 antibody as an active ingredient.

2. The therapeutic agent according to claim 1 in which the anti-IL-8 antibody is a monoclonal antibody.

3. The therapeutic agent according to claim 1 in which the anti-IL-8 antibody is an antibody against mammalian IL-8.

4. The therapeutic agent according to claim 3 in which the anti-IL-8 antibody is an antibody against human IL-8.

5. The therapeutic agent according to claims 2 to 4 in which anti-IL-8 antibody is WS-4 antibody.

6. The therapeutic agent according to claim 1 in which anti-IL-8 antibody is a humanized or chimeric antibody.

7. The therapeutic agent according to claim 2 to 6 in which anti-IL-8 antibody is a humanized WS-4.
